# EUROPEAN PATENT SPECIFICATION

(11) **EP 3 658 036 B1**
(45) Date of publication and mention of the grant of the patent: **13.09.2023**
(21) Application number: 18756031.3
(22) Date of filing: 27.07.2018
(51) Int. Cl.: A61B 8/00, A61B 17/34, A61B 18/14, A61M 25/00, A61B 8/12, A61B 8/08, A61M 25/06, A61M 25/10, A61M 29/00, A61B 17/00, A61B 17/22, A61B 90/00, A61B 18/00

(54) **DIRECTIONAL BALLOON TRANSSEPTAL INSERTION DEVICE FOR MEDICAL PROCEDURES**
VORRICHTUNG ZUR TRANSSEPTALEN EINFÜHRUNG EINES GERICHTETEN BALLONS FÜR MEDIZINISCHE VERFAHREN
DISPOSITIF D'INSERTION PAR VOIE TRANSSEPTALE DE BALLONNET DIRECTIONNEL POUR PROCÉDURES MÉDICALES

(30) Priority: 28.07.2017 US 201762538552 P; 29.11.2017 US 201762592061 P
(43) Date of publication of application: 03.06.2020
(73) Proprietor: East End Medical LLC, Lewes, DE 19958 (US)
(72) Inventor: MAINI, Brijeshwar S., West Palm Beach FL 33405 (US)
(74) Representative: Patentwerk B.V.
(86) International application number: PCT/US2018/044143
(87) International publication number: WO 2019/023609

(56) References cited:
- US-A- 5 135 001
- US-A- 5 135 001
- US-A- 5 295 958
- US-A- 5 865 801
- US-A- 5 865 801
- US-A- 6 102 907
- US-A1- 2004 215 233
- US-A1- 2014 309 675
- US-A1- 2014 309 675
- US-A1- 2015 217 093
- US-A1- 2016 051 321
- US-A1- 2016 051 321
- US-A1- 2017 105 761
- US-A1- 2017 135 559
- US-A1- 2017 135 559

## Description

### RELATED APPLICATIONS

The present invention claims priority on and from U.S. Provisional Application Serial No. 62/538,552, filed July 28, 2017 and Entitled "Directional Balloon Transseptal Insertion Device for Medical Procedures" and U.S. Provisional Application Serial No. 62/592,061, filed November 29, 2017 and Entitled "Directional Balloon Transseptal Insertion Device for Medical Procedures,"

### FIELD

The present invention relates generally to cardiac catheters, and more particularly, to a transseptal insertion device which is suitable for facilitating quick and safe transseptal puncture and insertion of a catheter through a cardiac septum to provide access to the left atrium in implementation of a left atrial intervention.

### BACKGROUND

Cardiac catheterization is a medical procedure in which a long thin tube or catheter is inserted through an artery or vein into specific areas of the heart for diagnostic or therapeutic purposes. More specifically, cardiac chambers, vessels and valves may be catheterized.

Cardiac catheterization may be used in procedures such as coronary angiography and left ventricular angiography. Coronary angiography facilitates visualization of the coronary vessels and finding of potential blockages by taking X-ray images of a patient who has received a dye (contrast material) injection into a catheter previously injected in an artery. Left ventricular angiography enables examination of the left-sided heart chambers and the function of the left-sided valves of the heart, and may be combined with coronary angiography. Cardiac catheterization can also be used to measure pressures throughout the four chambers of the heart and evaluate pressure differences across the major heart valves. In further applications, cardiac catheterization can be used to estimate the cardiac output, or volume of blood pumped by the heart per minute.

Some medical procedures may require catheterization into the left atrium of the heart. For this purpose, to avoid having to place a catheter in the aorta, access to the left atrium is generally achieved by accessing the right atrium, puncturing the interatrial septum between the left and right atria of the heart, and threading the catheter through the septum and into the left atrium. Transseptal puncture must be carried out with extreme precision, as accidental puncturing of surrounding tissue may cause very serious damage to the heart. In addition, transseptal puncture may require complicated instruments which are not helpful in guaranteeing the precision of the puncture.

The use of devices available today present many challenges for doctors attempting to puncture the interatrial septum and perform cardiac catheterization. Locating the interatrial septum, properly placing the distal end of the puncturing device at the desired location of the septum, safely puncturing the interatrial septum, avoiding accidental punctures, and tracking and maneuvering the catheter post-puncture, are among the many challenges facing those performing cardiac catheterization today. US 2014/309675 A1 discloses a transseptal insertion device comprising a sheath with a balloon and a dilator for puncturing the cardiac interatrial septum. US 2017/135559 A1 discloses a transseptal crossing device having an ultrasound imaging sensor on a catheter inside a balloon.

Accordingly, there is an established need for a device that is suitable for facilitating quick and safe transseptal puncturing to provide access to the left atrium in implementation of a left atrial intervention.

### SUMMARY

The invention is defined by claims 1 and 4. Further embodiments of the invention are defined by the dependent claims. Methods are not claimed.

Embodiments described herein overcome the disadvantages of the prior art. Embodiments provide for a device that is suitable for facilitating quick and safe transseptal puncturing to provide access to the left atrium in implementation of a left atrial intervention.

These and other advantages may be provided, for example, by a transseptal insertion device which is suitable for facilitating precise and safe transseptal puncture of a cardiac interatrial septum. The transseptal insertion device includes a sheath that defines at least one lumen therein and has a distal end that is closest to the cardiac interatrial septum of a patient when the transseptal insertion device is in use and a proximal end that is external to the patent, a balloon that is connected to the distal end of the sheath, in which the balloon, when inflated and the transseptal insertion device is in use, overhangs and extends past the distal end of the sheath, preventing accidental puncturing of the cardiac interatrial septum and stabilizing the transseptal insertion device against fossa ovalis of the cardiac interatrial septum, and a dilator that is positioned within the at least one lumen when the transseptal insertion device is in use. The dilator has a distal end and is designed to and is capable of precisely puncturing the cardiac interatrial septum without the use of a needle or other sharp instrument.

Embodiments may also overcome the disadvantages of the prior art and provide numerous advantages by including various features, including a dilator designed to remain sub-planar to the overhanging portion of the balloon until the dilator is extended to puncture the cardiac interatrial septum, the diameter of the lumen at rest being less than the diameter of the dilator, the sheath being made from malleable material capable of accommodating larger diameter devices within the lumen, the dilator including a dilator seal that is greater in diameter than the lumen and provides a water-tight seal of the lumen while the dilator seal remains within the lumen, sheath including inflation ports communicatively coupling the lumen to the balloon so that inflation liquid may be passed through lumen into the balloon, inflating the balloon, and the dilator seal being located on the distal end of the dilator such that moving the distal end of the dilator out of the lumen so that the dilator seal is external to the distal end of the sheath un-seals the lumen and causes the inflation liquid to flow out of the balloon and through the inflation ports, deflating the balloon. The transseptal insertion device may also include an energy source, such as a radio-frequency (RF) energy source, external to the proximal end of the sheath and operatively connected to the distal end of the dilator to deliver energy to the distal end of the dilator. The dilator may be designed to and is capable of precisely puncturing the cardiac interatrial septum using the energy, such as RF energy delivered to the distal end of the dilator. The transseptal insertion device of may also include an energy source external to the proximal end of the sheath and operatively connected to the balloon to deliver energy to the balloon and an external stabilizer connected to the proximal end of the sheath that stabilizes the transseptal insertion device and substantially prevents unwanted movement of the transseptal insertion device.

These and other advantages may be provided, for example, by an example method of precisely and safely transseptal puncturing a cardiac interatrial septum. The method may include inserting a transseptal insertion device into a right atrium of a patient's heart, inflating the balloon, in which the inflated balloon overhangs and extends past the distal end of the sheath and the dilator is sub-planar to the balloon overhang, preventing accidental puncturing of the cardiac interatrial septum, extending the distal end of the sheath so that the inflated balloon is positioned against the fossa ovalis of the cardiac interatrial septum at a desired puncture point, thereby stabilizing the transseptal insertion device against fossa ovalis, extending the distal end of the dilator past the balloon overhang, puncturing the cardiac interatrial septum with the dilator without the use of a needle or other sharp instrument. Puncturing the cardiac interatrial septum with the dilator may include applying RF energy through the dilator to the cardiac interatrial septum. Extending the distal end of the dilator past the balloon overhang may cause the balloon to deflate. The method may include extending the distal end of the sheath past the cardiac interatrial septum and re-inflating the balloon.

These and other advantages may be provided, for example, by a transseptal insertion device which is suitable for facilitating precise and safe transseptal puncture of a cardiac interatrial septum. The transseptal insertion device may include a sheath that defines a lumen therein and has a distal end that is closest to the cardiac interatrial septum of a patient when the transseptal insertion device is in use and a proximal end that is external to the patent, the sheathe including inflation ports near the distal end that enable inflation fluid in the lumen to exit and enter the sheath. The device may further include a balloon that is connected to the distal end of the sheath, in which the balloon, when inflated and the transseptal insertion device is in use, overhangs and extends past the distal end of the sheath, preventing accidental puncturing of the cardiac interatrial septum and stabilizing the transseptal insertion device against fossa ovalis of the cardiac interatrial septum and the balloon is positioned over the inflation ports of the sheath so that inflation fluid may exit the sheath and enter the balloon, inflating the balloon, and exit the balloon and enter the sheath, deflating the balloon. The device may further include a dilator that is positioned within the lumen when the transseptal insertion device is in use. The dilator has a distal end and includes a dilator seal at the distal end that occludes the lumen when the dilator is sub-planar to the balloon overhang so that the balloon remains inflated after inflation fluid is passed through the lumen into the balloon and automatically deflates when the distal end of the dilator is extended sufficiently past the distal end of the sheath so that the dilator seal does not occlude the lumen.

These and other advantages may also be provided, for example, by a transseptal insertion device which is suitable for facilitating precise and safe transseptal puncture of a cardiac interatrial septum. The transseptal insertion device includes a sheath that defines at least one lumen therein and has a distal end that is closest to the cardiac interatrial septum of a patient when the transseptal insertion device is in use and a proximal end that is external to the patent, a balloon that is connected to the distal end of the sheath and a dilator that is positioned within the at least one lumen when the transseptal insertion device is in use, in which the dilator has a distal end and is designed to and is capable of precisely puncturing the cardiac interatrial septum. The balloon, when inflated and the transseptal insertion device is in use, overhangs and extends past the distal end of the sheath, preventing accidental puncturing of the cardiac interatrial septum and stabilizing the transseptal insertion device against fossa ovalis of the cardiac interatrial septum and includes one or more ultrasound transceivers that emit and receive ultrasound waves, convert the ultrasound waves to electrical signals, and transmit the electrical signals to an external imaging device that produces images of the cardiac interatrial septum from the received signal. The one or more ultrasound transceivers may be located on the balloon surface or inside the balloon, may be oriented towards the cardiac interatrial septum when the balloon is fully inflated and the distal end of the sheath is oriented towards the cardiac interatrial septum, may be oriented perpendicular to the sheath when the balloon is deflated, may be configured in the shape of a cross so that one or more ultrasound transceivers are oriented towards the cardiac interatrial septum when the balloon is fully inflated and the distal end of the sheath is oriented towards the cardiac interatrial septum and perpendicular to the sheath, may be configured in the shape of a disc, may be connected to an external imaging device through a wire that runs via lumen in sheath or to an external imaging device via WiFi or other wireless connection.

These and other advantages may also be provided, for example, by a transseptal insertion device which is suitable for facilitating precise and safe transseptal puncture of a cardiac interatrial septum. The transseptal insertion device includes a sheath that defines at least one lumen therein and has a distal end that is closest to the cardiac interatrial septum of a patient when the transseptal insertion device is in use and a proximal end that is external to the patent, a balloon that is connected to the distal end of the sheath, in which the balloon, when inflated and the transseptal insertion device is in use, overhangs and extends past the distal end of the sheath, preventing accidental puncturing of the cardiac interatrial septum and stabilizing the transseptal insertion device against fossa ovalis of the cardiac interatrial septum, and a dilator that is positioned within the at least one lumen when the transseptal insertion device is in use, in which the dilator has a distal end and is designed to and is capable of precisely puncturing the cardiac interatrial septum. The sheath includes one or more ultrasound transceivers that emit and receive ultrasound waves, convert the ultrasound waves to electrical signals, and transmit the electrical signals to an external imaging device that produces images of the cardiac interatrial septum from the received signals. The ultrasound transceivers may be oriented towards the distal end of the sheath or towards the proximal end of the sheath.

These and other advantages may also be provided, for example, by a transseptal insertion device which is suitable for facilitating precise and safe transseptal puncture of a cardiac interatrial septum. The transseptal insertion device includes a sheath that defines at least one lumen therein and has a distal end that is closest to the cardiac interatrial septum of a patient when the transseptal insertion device is in use and a proximal end that is external to the patent, a balloon that is connected to the distal end of the sheath, and a dilator that is positioned within the at least one lumen when the transseptal insertion device is in use, in which the dilator has a distal end and is designed to and is capable of precisely puncturing the cardiac interatrial septum. The balloon, when inflated and the transseptal insertion device is in use, overhangs and extends past the distal end of the sheath, preventing accidental puncturing of the cardiac interatrial septum and stabilizing the transseptal insertion device against fossa ovalis of the cardiac interatrial septum and is constructed of compliant and non-compliant material so that different portions of the balloon will expand differentially when the balloon is inflated. The transseptal insertion device may also include one or more ultrasound transceivers that emit and receive ultrasound waves, convert the ultrasound waves to electrical signals, and transmit the electrical signals to an external imaging device that produces images of the cardiac interatrial septum from the received signals.

These and other advantages may also be provided, for example, by a transseptal insertion device which is suitable for facilitating precise and safe transseptal puncture of a cardiac interatrial septum. The transseptal insertion device includes a sheath that defines at least one lumen therein and has a distal end that is closest to the cardiac interatrial septum of a patient when the transseptal insertion device is in use and a proximal end that is external to the patent, a balloon that is connected to the distal end of the sheath, in which the balloon, when inflated and the transseptal insertion device is in use, overhangs and extends past the distal end of the sheath, preventing accidental puncturing of the cardiac interatrial septum and stabilizing the transseptal insertion device against fossa ovalis of the cardiac interatrial septum, a dilator that is positioned within the at least one lumen when the transseptal insertion device is in use, in which the dilator has a distal end and a lumen defined therein, and a needle with a blunt end that is positioned within the lumen of the dilator and is designed to and is capable of precisely puncturing the cardiac interatrial septum with application of energy transmitted through the needle, in which the needle includes malleable and rigid portions. The needle may include a distal end and proximal end of the needle. The energy applied by the needle may be radio-frequency (RF) energy.

These and other obj ects, features, and advantages of embodiments of the present invention will become more readily apparent from the attached drawings and the detailed description, which follow.

### Brief Description of the Drawings

The preferred embodiments described herein and illustrated by the drawings hereinafter be to illustrate and not to limit the invention, where like designations denote like elements, and in which:
FIG. 1A is a front perspective, cross-sectional view of an embodiment of a transseptal insertion device.
FIG. 1B is a front perspective, cross-sectional view of an embodiment of a transseptal insertion device showing a dilator extending partially through and extending out from device.
FIG. 1C is a front perspective, cross-sectional view of an embodiment of a transseptal insertion device showing a dilator extending partially through the device.
FIG. 2 is a front perspective, cross-sectional view of an embodiment of a transseptal insertion device showing inflated overhanging balloon and dilator positioned within device and subplanar to overhanging balloon.
FIG. 3 is a cross-sectional, end view of an embodiment of a transseptal insertion device and dilator shown prior to puncturing an interatrial cardiac septum with inflated overhanging balloon.
FIG. 4 is a front perspective, cross-sectional view of an embodiment of a transseptal insertion device with dilator advanced forward in order to tent an interatrial septum.
FIG. 5 is a front perspective, cross-sectional view of an embodiment of a transseptal insertion device with a transseptal wire advanced post-puncture through interatrial septum.
FIGS. 6A-6C are front perspective, cross-sectional views of an embodiment of a flexible transseptal insertion device with different angulations
FIG. 7 is a front perspective, cross-sectional view of an embodiment of a transseptal insertion device with radiofrequency energy capability.
FIG. 8 is a side view of an embodiment of transseptal insertion device with an overhanging balloon with marking.
FIG. 9 is a side view of an embodiment of transseptal insertion device with an overhanging balloon with a marker band.
FIG. 10 is a cross-sectional side view of an embodiment of a transseptal insertion device that includes a dilator with an electrode tip
FIG. 11A is a side view of an embodiment of a dilator that may be used in embodiments of a transseptal insertion device.
FIG. 11B is a side view of a distal end of an embodiment of a dilator that may be used in embodiments of a transseptal insertion device.
FIG. 12A and 12B are side views of an embodiment of a transseptal insertion device, and interatrial septum, that includes a dilator with an ablation tip.
FIG. 13 is a side view of an embodiment of a transseptal insertion device with mechanical deflection capability.
FIG. 14 is side views of embodiments of curved dilators that may be used in embodiments of a transseptal insertion device.
FIG. 15 is a cross-sectional, side view of an embodiment of a steerable transseptal insertion device.
FIG. 16 is a perspective side view of a proximal end of an embodiment of a transseptal insertion device showing a handle and a stabilizer.
FIGS. 17A and 17B are side views of an embodiment of a transseptal insertion device with balloons capable of differential inflation.
FIG. 18 is a side view of a malleable or flexible transseptal needle that may be used in embodiments of a flexible transseptal insertion device with multiple angulations.
FIGS. 19A-19B are side views of embodiments of transseptal insertion device with ultrasound imaging or visualizing capability.

### Detailed Description

The following detailed description is merely exemplary in nature and is not intended to limit the described embodiments or the application and uses of the described embodiments. As used herein, the word "exemplary" or "illustrative" means "serving as an example, instance, or illustration." Any implementation described herein as "exemplary or "illustrative" is not necessarily to be construed as preferred or advantageous over other implementations. All of the implementations described below are exemplary implementations provided to enable persons skilled in the art to make or use the embodiments of the disclosure and are not intended to limit the scope of the disclosure, which is defined by the claims. Furthermore, there is no intention to be bound by any expressed or implied theory presented in the preceding technical field, background, brief summary or the following detailed description. It is also to be understood that the specific devices and processes illustrated in the attached drawings, and described in the following specification, are simply exemplary embodiments of the inventive concepts defined in the appended claims. Hence, specific dimensions and other physical characteristics relating to the embodiments disclosed herein are not to be considered as limiting, unless the claims expressly state otherwise.

Embodiments of the present invention are directed to a transseptal insertion device or catheter which is suitable for facilitating quick and safe transseptal insertion of a needle or catheter through an interatrial cardiac septum/septal wall to provide access to the left atrium in implementation of a left atrial intervention. The transseptal insertion device is elongated yet has a relatively reduced length, and can be easily and safely turned within an atrium of the heart to achieve a correct orientation towards the cardiac septum.

In a first implementation, an embodiment includes a transseptal insertion device which is suitable for facilitating a precise and safe transseptal insertion of a needle, wire, dilator or catheter through a cardiac septum. The transseptal insertion device includes an overhanging balloon that, e.g., measures 2-12 mm and is either air or fluid filled when inflated. The dilator is sub-planar to the balloon at the time of contact with the atrial tissue (e.g., the interatrial septum).

In operation of an embodiment, once the balloon is noted to tent the interatrial septum, the dilator is advanced to a pre-specified position which is controlled by the movement of the dilator, which results in further tenting of the septum. Once it is confirmed that the tenting of the interatrial septum is accurate, the transseptal needle may be advanced across the septum. Following this the balloon may be deflated or may be kept inflated for more stability and for purpose of preventing the sheath from advancing too deep into the left atrium.

In another embodiment, the transseptal insertion device may include multiple dilators that may be inserted into the transseptal insertion device sheath. Each dilator may be pre-configured at a different angle so that, when inserted at in the sheath, the dilator will cause the transseptal insertion device to bend or flex so that the angle of a dilator to the interatrial septum to vary from 0-270°. This allows the transseptal insertion device to target different areas of the left atrium and parts of the heart accessible through the left atrium (*e.g.*, the pulmonary veins, left atrial appendage, mitral valve, or the left ventricle). Each of these different areas of parts may require a different puncture/insertion point through the septum wall and a different angle of puncture and angle of approach in the left atrium. The pre-configured dilators enable the desired angulation to be chosen and effectuated.

Another embodiment of the transseptal insertion device may include an actuator which allows flexion and extension of the catheter and the transseptal sheath, which would, therefore, allow for motion of the transseptal sheath in all three-dimensional planes (*i.e.,* x, y, z) and in both directions in each plane (*e.g.,* up and down, left and right, etc.). In embodiments, the transseptal sheath used may have a variety of diameters or gauges. For example, the French (Fr) size or gauge of the transseptal sheath may range from 6-40 Fr in embodiments. The different gauge allows different size devices to be inserted through the transseptal insertion device.

Additionally, the dilator tip may house a cap, crown, or electrode tip that is connected to a thermal, laser, sonic, electrical, or radiofrequency energy source via the dilator at an exterior hub connected to the dilator through the proximal end of the transseptal insertion device. Accordingly, the dilator tip may deliver energy to the septum to create a safe and controlled puncture. In this manner, embodiments avoid the need to use needles and the disadvantages inhered therein. As noted, the types of energy delivered could be heat, cold, laser, sonic, electrical, or radiofrequency.

In an embodiment that includes a sheath dilator capable of transmitting energy, there is no need for a sharp, metallic transseptal needle. The dilator itself therefore may be moved across the interatrial septum and the lumen of the dilator then be used to pass a variety of wires or other tools into the left atrium. The lumen of the dilator may also be used for pressure monitoring and assessment of blood oxygen saturation through various sensors deployed in the lumen. For example, various devices may be attached to the dilator through an external hub located at the proximal end of the transseptal insertion device. Such devices may include a fluid-filled pressure transducer to measure pressure, *e.g*., in the atrial chamber, a solid-state sensor for measuring pressure, oximetry, or other characteristics of the atrial chamber, and/or a device for drawing fluid (*e.g.,* blood) from the atrium for testing.

In an embodiment in which the dilator carries energy, the dilator may include a wire that may be energized by the dilator. The wire may be advanced into the left atrium to deliver energy to puncture the left atrial septum. The wire may be, for example, a wire with a 0.0014 or larger diameter. Such wires would significantly decrease the footprint of the device entering the left atrium.

In an embodiment, the sheath balloon is inflated using air or fluid via the sheath and there is no separate port or hypotube for inflation or deflation of the balloon. Such embodiments may include inflation ports in the sheath and a dilator seal on the dilator. The inflation ports may be implemented as multiple holes in the transseptal insertion device sheath that permit inflation fluid or gas to flow through a lumen in the sheath to the balloon and back out again when unsealed by the moving dilator seal

The dilator seal may be a bump or ridge located near the distal end of the dilator. When the dilator seal is covered by the overhanging sheath balloon, the seal is closed and the balloon may remain inflated. As the dilator is advanced beyond the overhanging balloon, the dilator seal moves beyond the seal zone, the dilator seal is opened or unsealed, resulting in the balloon automatically deflating. Prior art mechanisms require multiple parts on two different components that move relative to one another to seal and unseal an inflation chamber. The present embodiment requires only the ridge on the dilator.

When the balloon deflates, the inflation liquid or gas, which is inert, flows out of the balloon and into the heart, through which it is absorbed into the blood. The inflation air or fluid may include contrast agents enabling easier detection of inflation and deflation using imaging.

Embodiments may include radiopaque and echogenic markers on the balloon and the dilator. These markers may be in the form of letters, such as an E or a C. These markers allow for the appropriate positioning of the catheter in the 3-dimensional space (*e.g.*, of the atrium) using imaging to view the markers and, therefore, the position of the balloon and dilator.

Embodiments may include a ring or band in the middle of the balloon. The ring in the middle of the balloon is for same purpose as the letter markings above - namely, to act as a navigational guide.

Various embodiments as described above are illustrated by the drawings. Shown throughout the drawings, embodiments of the present invention is directed toward a transseptal insertion device which is suitable for facilitating quick and safe transseptal puncturing of an interatrial septum and insertion of a catheter there thru to provide access to the left atrium in implementation of a left atrial intervention. Embodiments are suitable for other uses as well. Referring to the drawings described below, exemplary embodiments of a transseptal insertion device are illustrated. Together, the drawings illustrate method of using transseptal insertion device to puncture interatrial cardiac septum and extend and insert catheter (or other component inserted through transseptal insertion device) across interatrial septum and into left atrium. As shown, the transseptal insertion device is generally elongated and arranged along a longitudinal axis.

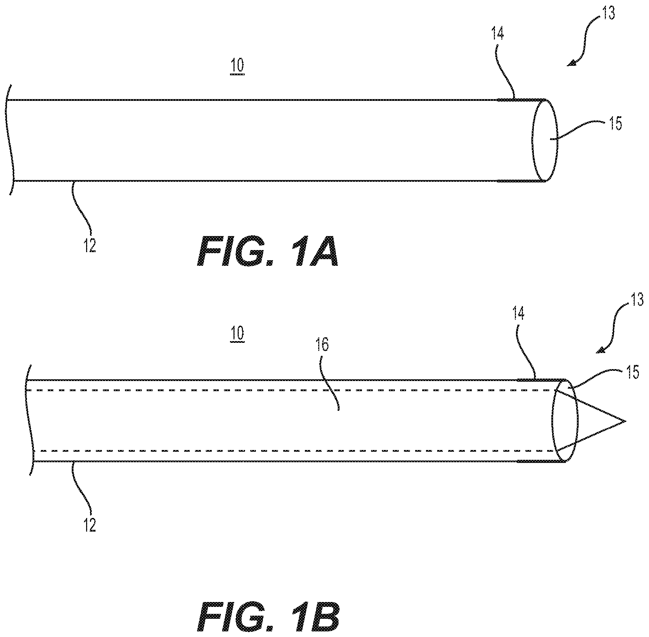

With reference now to FIGS. 1A-1C, shown is an embodiment of transseptal insertion device or catheter **10.** Shown is the distal end of transseptal insertion device **10**, *i.e.,* the end of device **10** with opening through which dilator, catheter, and needle may extend, *e.g*., to puncture interatrial cardiac septum. As shown in FIG. 1A, transseptal insertion device **10** includes outer sheath or balloon shaft **12** and balloon **14** located at distal tip **13** of transseptal insertion device **10.** Sheath **12** may contain and define a center lumen **15.** Sheath **12** may be fabricated from various materials, including, *e.g.*, polymers, including thermoplastics elastomers (TPEs) such as PEBA (*e.g*., Pebax^{®}), nylons, thermoplastic polyurethanes (TPUs) such as Pellathane^{®}, similar materials and combinations thereof. Sheath **12** may be referred to as catheter and used in cardiac catheterizations. After puncture, sheath **12** may be inserted through septum into left atrium. Alternatively, sheath **12** may contain a separate catheter that is inserted through septum post puncture.

Transseptal insertion device **10** also includes dilator **16,** positioned in center lumen **15,** as shown in FIG. 1B. Balloon **14** is preferably sealed, air-tight and water-tight, on both its ends to sheath **12,** with openings or one or more inflation holes (not shown) in balloon **14** that open to lumen **15.** Connected to sheath **12** as such, inflation liquid or gas may be passed through inflation holes into balloon **14,** inflating balloon **14.** Lumen **15** is kept sealed by dilator seal (not shown) that forms air and water tight seal of lumen **15** (until dilator seal on dilator **16** passes distal tip **13** and exits lumen **15**).

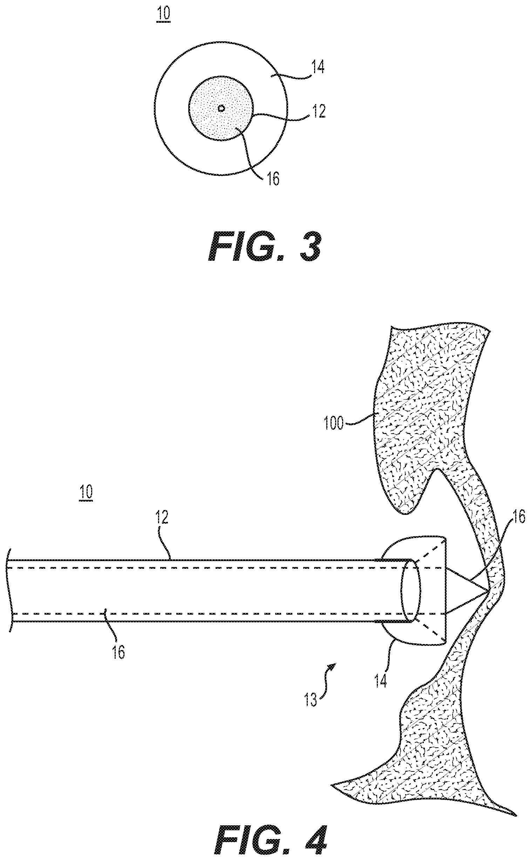

With continuing reference to FIG. 1A, in view shown, overhanging balloon **14** is uninflated. Although cross-section of balloon **14** is shown on top and bottom of distal tip **13,** balloon **14** preferably extends around circumference of distal tip or end **13** of transseptal insertion device **10** (see FIG. 4). Overhanging balloon **14** is of form such that balloon **14** overhangs or extends from distal tip **13** of sheath **12** when inflated.

In FIG. 1B, dilator **16** is shown positioned within and partially extending out of sheath **12,** past distal tip **13** of device **10.** Overhanging balloon **14** is uninflated and dilator **16** extends past balloon **14.** It is noted that the relative sizes of sheath **12** and dilator **14** shown are for illustrative purposes as the diameter of dilator **14** may be relatively larger or smaller than shown in relation to the diameter of sheath **12,** although dilator **14** necessarily has a smaller diameter than sheath **12.** Although dilator **14** is shown to have a pointed end, dilator **14** may have a rounded or relatively flat end. Embodiments, as described herein, are designed and intended to puncture septum without use of a needle or other sharp instrument.

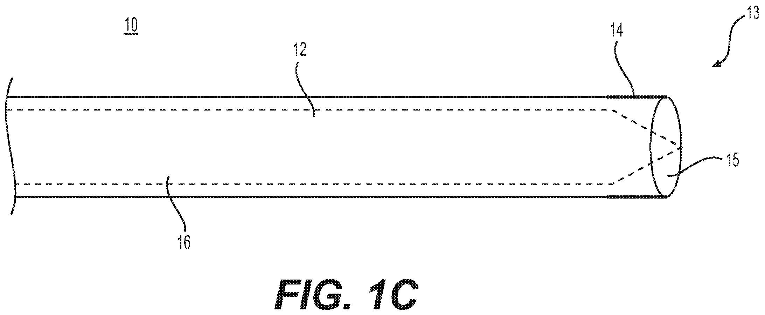

With reference now to FIG. 1C, dilator **16** is shown positioned within center lumen **15** of sheath **12.** Tip of dilator **16** is positioned within distal tip **13** of transseptal insertion device **10** sub-planar to end of transseptal insertion device **10.** The position shown is position dilator **16** may be in immediately prior to inflation of balloon **14.** It is noted that the relative sizes of catheter/sheath **12** and dilator **16** shown are for illustrative purposes as the diameter of dilator **16** may be relatively larger or smaller than shown in relation to the diameter of sheath **12.** Ordinarily, dilator **16** has smaller diameter or gauge then catheter/sheath **12,** although fit of dilator **16** in catheter/sheath **12** is preferably snug enough so that dilator **16** does not move (laterally or axially) relative to position or "wobble" within transseptal insertion device **10.** catheter **18** necessarily has a smaller diameter than sheath **12.** In embodiments, sheath **12** material may be sufficiently malleable to enable larger diameter dilators **16,** and other larger diameter devices, to be passed through sheath **12.** In such embodiments, the sheath **12** will stretch to accommodate the larger diameter dilator **16** or other device.

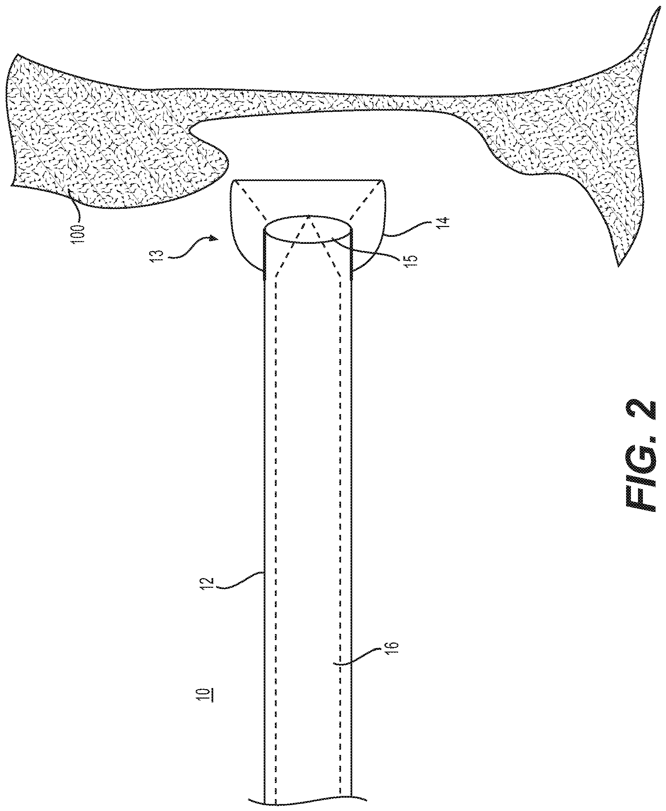

With reference now to FIG. 2, shown is distal end of an embodiment of transseptal insertion device **10** in which overhanging balloon **14** is inflated. Dilator **16** is shown positioned within center lumen **15** of sheath **12** with tip of dilator **16** positioned at distal tip **13** of transseptal insertion device **10** and sub-planar to overhanging balloon **14.** The plane that is referred to here is the plane X-X, perpendicular to the axis of transseptal insertion device **10** and dilator **16,** formed by the end of overhanging balloon **14.** Hence, dilator **16** remains sub-planar to overhanging balloon **14** until operator intends balloon **14** to be deflated and dilator **16** to tent and puncture interatrial septum **100.** As noted above, balloon **14** preferably extends completely around circumference of tip **13** of transseptal insertion device **10.** Accordingly, FIG. 3 only illustrates cross-section of inflated balloon **14.**

With reference now to FIG. 3, shown is a perspective, cross-sectional view of distal end an embodiment of transseptal insertion device **10** in which overhanging balloon **14** is inflated. As shown, inflated overhanging balloon **14** preferably extends around entire circumference of sheath **12** (and, therefore, device **10)**. Shown situated within lumen **15** of sheath **12** is tip of dilator **16.** Tip of dilator **16** is positioned within tip **13** of transseptal insertion device **10,** as it would be prior to being extended past tip **13** and puncturing an interatrial cardiac septum.

With reference now to FIG. 4, shown is shown is distal end of an embodiment of transseptal insertion device **10** with dilator **16** advanced forward in order to tent the interatrial septum **100.** Dilator **16** is shown extending through center lumen **15** of sheath **12** and past overhanging balloon **14.** Since dilator **16** is no longer sub-planar to overhanging balloon **14** and has moved past overhanging balloon **14,** dilator seal (not shown) has moved and unsealed balloon **14,** causing it to begin to deflate. Extended as such, and pressed against interatrial septum **100,** dilator **16** tents the interatrial septum **100** away from transseptal insertion device **10.**

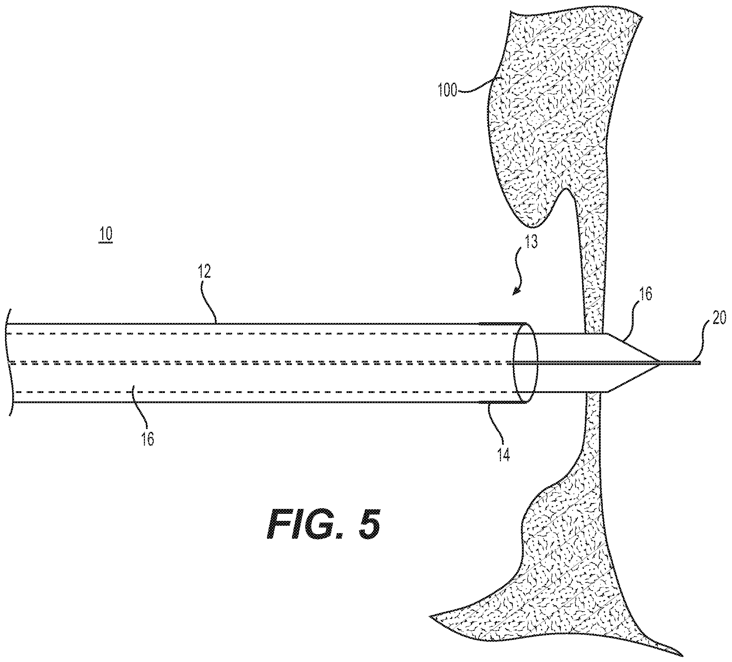

With reference now to FIG. 5, shown is shown is distal end of an embodiment of transseptal insertion device **10** with dilator **16** advanced forward through interatrial septum **100,** after puncturing septal wall (*e.g*., through application of energy through dilator **16** as described herein) and transseptal wire or wire rail **20** extending through dilator **16** and into left atrium chamber **110.** Wire rail **20** may sit in lumen **19** of dilator **16.** Dilator **16** may be used as a conduit to advance the wire rail **20** into the left atrium.

Wire rail **20** may act as a guide for devices to enter the left atrium through the puncture in the septal wall made by transseptal insertion device **10.** For example, wire rail may guide transseptal insertion device **10** or other catheters in the left atrium. In this manner, catheters may be advanced safely into the left atrium over or guided by wire rail **20.** In an embodiment, wire rail **20** may be energized (*e.g.*, to ablate or puncture the septum with energy delivered from source at proximal end of transseptal insertion device **10).**

With continued reference to FIG. 5, dilator **16** preferably defines and includes an opening or lumen **19** extending through its tip and through which transseptal wire **20** extends. With dilator **16** extended as shown and tenting interatrial septum, septum may be punctured by energy delivered through cap or electrode at tip of dilator **16** (see below) and transseptal wire rail **20** extended through opening in tip of dilator **16** and through puncture made in interatrial septum by dilator **16** cap.

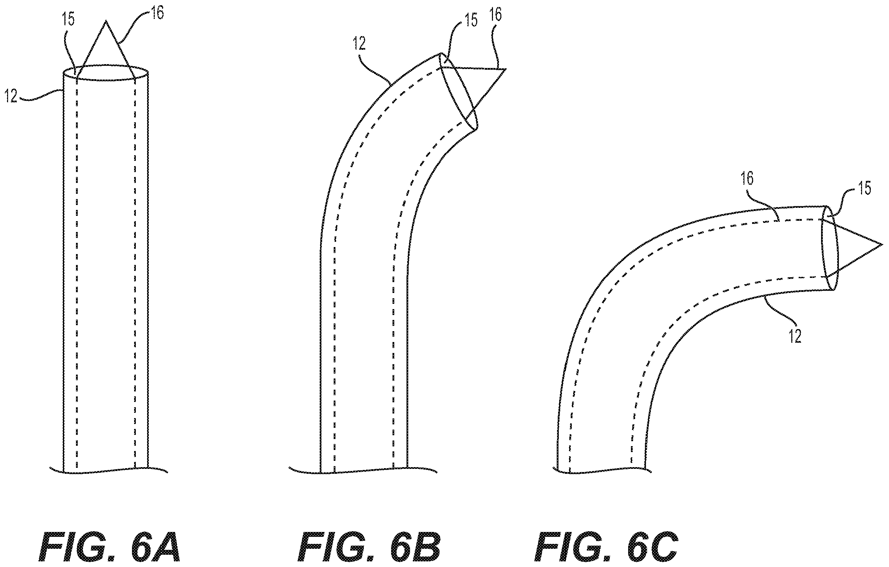

With reference to FIGS. 6A-6C, shown are different views of an embodiment of transseptal insertion device **10** with a flexible sheath **12** flexed or angulated at different angles. Transseptal insertion device **10** may be flexed or angulated depending on the anatomy of the atria using fixed angled dilators **16** that are inserted into lumen **15** of sheath **12,** causing sheath **12** to flex. Such fixed angled dilators **16** may be, *e.g*., any angle from 0-270°. Alternatively, sheath **12** and dilator **16** may be both flexible (preferably, needle and catheter inserted through such flexible sheath **12** are flexible or malleable, at least in part) and transseptal insertion device **10** may be flexed or angulated, thereby flexing or angulating sheath **12** and dilator **16,** using, *e.g*., a handle or wire (not shown) connected to tip **13** of device **10.** Handle and/or wire may also be used to turn or flex or move tip **13** of transseptal insertion device **10,** *e.g*., moving tip **13** of sheath "up" or "down" or "left" or "right" or angulating tip **13** relative to axis of sheath **12** as shown.

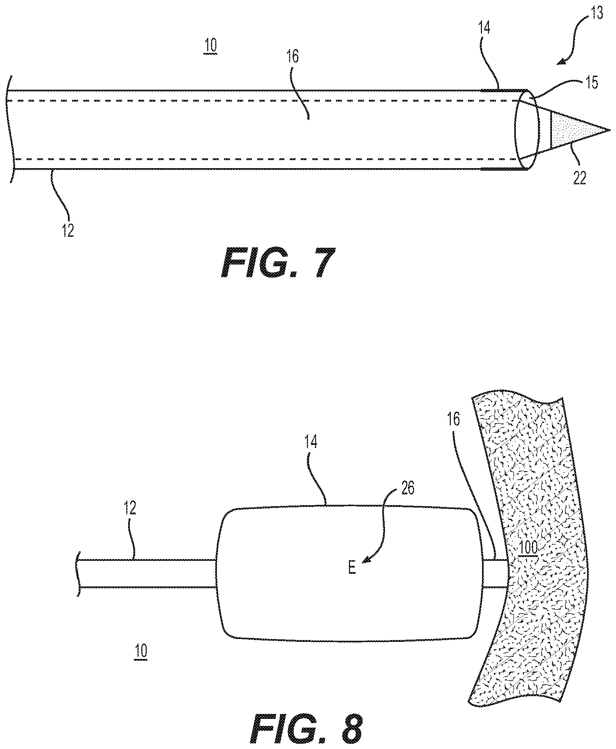

With reference now to FIG. 7, shown is an embodiment of transseptal insertion device **10** with radiofrequency energy capability. Transseptal insertion device **10** shown includes sheath **12,** overhanging balloon **14,** and dilator **16.** Dilator **16** may include cap or crown **22,** on distal end as shown, with RF energy capability or capable of delivering RF energy. Alternatively, cap or crown may include or be an RF electrode. Dilator **16** may be connected, *e.g.*, on proximate end (not shown) to a radiofrequency (RF) energy source (not shown) at, *e.g*., external hub, that provides RF energy to cap or crown **22.** The RF energy may be delivered through dilator **16.** So equipped with cap or crown **22,** dilator **16** may tent interaxial septum and create puncture of interaxial septum through delivery of RF energy. In this embodiment, the use of a sharp needle may be avoided.

As described above, embodiments may be capable of delivering other energy, such as thermal, laser, sonic, or electrical energy for the purposes of puncturing the septum. Such embodiments may be constructed in a similar manner, with dilator or needle including cap or crown at a distal end capable of delivering thermal, laser, sonic, or electrical energy, and such energy may be delivered through dilator or needle connected, *e.g*., on proximate end (not shown) to a thermal, laser, sonic, or electrical energy source (not shown) at, *e.g*., external hub. So connected, dilator or needle may use thermal, laser, ultrasound, or electrical energy to puncture interaxial septum, avoiding the need for a sharp needle.

A significant challenge for operators of transseptal devices today is the difficulty in determining how posterior (towards the back of a patient) the transseptal device is located. The left atrium is on the posterior side of the heart. It is, therefore, often critical to be able to determine how posterior is the distal tip **13** of transseptal insertion device **10** in order to successfully locate the interatrial septum. Generally tracking the location of the distal end of transseptal insertion device **10** is critical to safe operation. With reference now to FIG. 8, shown is distal end of an embodiment of transseptal insertion device **10** with inflated overhanging balloon **14.** Balloon **14** shown is an embodiment with one or more markers **24.** Marker **24** may be, *e.g*., a radiopaque and/or echogenic marker **24.** As a radiopaque or echogenic marker, marker **24** will be visible on scanners used by those performing cardiac catheterizations. The markers **24** may be in the form of letters, such as an E or a C. Marker **24** enables the appropriate positioning of balloon **14** and catheter **18** in the 3-dimensional space (*e.g.,* of the atrium) using imaging to view the marker **24** and, therefore, the position of balloon **14.**

Specifically, in operation, the less posterior distal tip **13** is positioned, the more of the E (or C) will be shown. As operator of transseptal insertion device **10** turns or rotates distal tip **13** toward posterior of patient, less of the arms of the E will be seen. In a preferred embodiment, when only the vertical portion of the E is visible (*i.e.*, appearing as an I) distal tip **13** will be rotated to its maximum posterior position. Consequently

With continuing reference to FIG. 8, balloon **14** is shown as inflated. However, distal end of dilator **16** is shown extruding or extending distally from balloon **14,** past plane formed by distal end of inflated balloon **14.** According, dilator **16** has been moved into the tenting and puncturing position, adjacent to interaxial septum, dilator seal (not shown) has exited or soon will exit sheath **12,** balloon **14** is deflating or will soon deflate, and puncture of the interaxial septum is imminent.

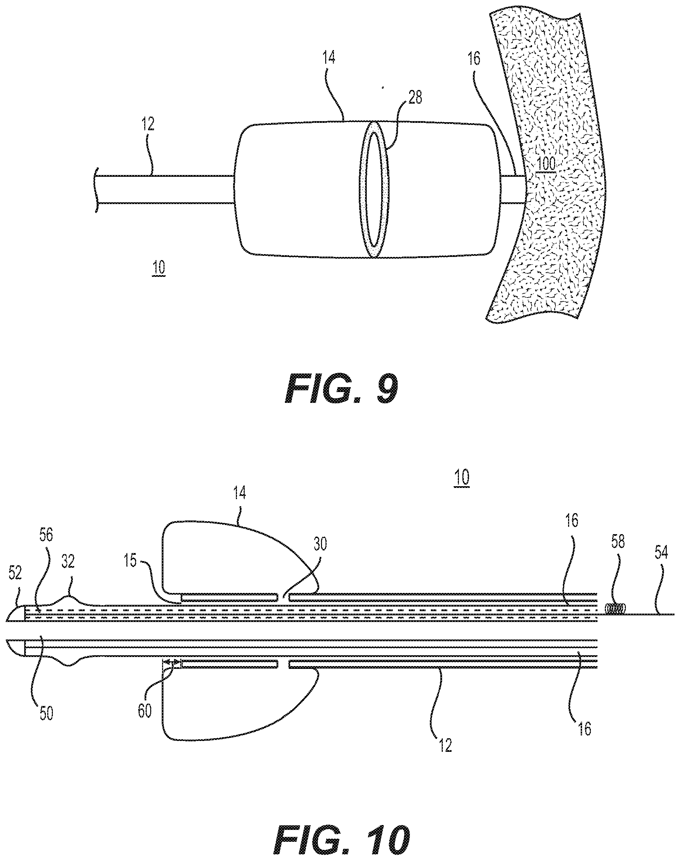

With reference now to FIG. 9, shown is another embodiment of overhanging balloon **14** which may be deployed in embodiments of transseptal insertion device **10.** Overhanging balloon **14** may include ring or band **28** around a portion of balloon **14.** Ring or band **28** may serve as a marker, similar to markers **24** shown in FIG. 8. Hence, ring **28** may be radiopaque or echogenic and may be view by scanning devices used for visualization in cardiac catheterizations (*e.g.*, fluoroscopic imaging devices). Similar to the letter E or C, the view of the ring **28** changes as the distal tip **13** of transseptal insertion device **10** moves more posterior. When in a least posterior position, ring **28** may appear as just a line or band positioned across axis of transseptal insertion device **10.** When device **10** is rotated so that distal tip **13** is significantly closer to the posterior, ring **28** may appear as a full "flat" circle or ring. In FIG. 8, distal tip **13** is partially rotated so that ring **28** is partially visible.

With reference to both FIGS. 8 and 9, the marker **24** and ring **28** are described and shown as located on balloon **14.** In embodiments, marker **24** and/or ring **28** may also be located on sheath **12** and/or dilator **16.** So located, marker **24** and/or ring **28** would operate in effectively the same manner as described above (*i.e.,* the arms of the E would disappear as the distal end was moved more to the posterior and the ring would become more visible). Markers **24** and/or rings **28** may be placed on all three of balloon **14,** sheath **12,** and dilator **16,** or a combination thereof.

With reference now to FIG. 10, shown is distal end of an embodiment of transseptal insertion device **10** that includes dilator **16** with electrode tip. Shaft of dilator **16** defines and contains a center lumen **50.** Lumen **50** may be defined in the range of, but not limited to, .020"-.040". Dilator **16** may be made from a polymer material (*e.g*., HDPE, LDPE, PTFE, or combination thereof). Dilator shaft **16** shown includes a distal electrode tip **52.** Electrode tip **52** may be comprise a metallic alloy (*e.g*., PtIr, Au, or combination thereof). In preferred embodiments, the size and shape of electrode tip **52** is selected to be sufficient to generate a plasma for *in vivo* ablation of tissue in an applied power range of, but not limited to, 20-30W. Electrical conductor **54** extends from electrode tip **52** to the proximal end (not shown) of the dilator **16.** Electrical conductor **54** may run axially through an additional lumen **56** defined by and contained in dilator shaft **16.** Electrical conductor **54** may contain a coil feature **58** to accommodate lengthening during bending or flexing of dilator **16.**

Dilator **16** may also include a distal dilator seal **32** for occlusion of sheath shaft **12** center lumen **15.** Dilator seal **32** may be a ring that extends around entire circumference of dilator **16.** Dilator seal **32** may be in the range of, but not limited to, 0.000"-0.005" larger in diameter than center lumen **15**. (1 inch = 2.54 cm). Distal seal **32** occludes lumen **15,** forming a liquid and gas tight seal so that balloon **14** remains inflated, until distal seal **32** exits lumen **15.** Attached to distal end of sheath **12** is contains overhanging balloon **14.** Overhanging balloon **14** may be made from a polymer material (*e.g.*, PET, Nylon, Polyurethane, Polyamide, or combination thereof). Overhanging balloon **14** may be in the range of, but not limited to, 5-20mm in diameter and 20-30mm in length. Overhanging balloon **14** may be inflated via injection of fluid (or gas) from the proximal end of sheath **12** center lumen **15** while distal dilator seal **32** occludes center lumen **15** distal to inflation ports **30** in sheath **12.** Inflation ports **30** are preferably defined flush to surface of sheath **12** and communicate with lumen **15** (*e.g.,* inflation ports may simply be holes defined in sheath that connect lumen to exterior of sheath). Balloon **14** is preferably connected to sheath **12** so that inflation ports **30** communicate with interior of balloon **14** and provide pathway for inflation fluid or gas to enter and inflate balloon **14** (and exit and deflate balloon **14**). During the proper functioning or operation of transseptal insertion device **10** for puncturing the interatrial septum, balloon **14** is deflated when dilator **16** moves out of lumen **15** and dilator seal **32** moves distal and outside of sheath **12.** However, deflation of overhanging balloon **14** may occur either via positioning of dilator seal **32** proximal to inflation ports **30** or distal and outside of sheath **12.** Overhanging balloon **14** is of form such balloon **14** overhangs or extends from distal end **13** of sheath **12.** Overhang or extension **60** may be in the range of, but not limited to, 0.0mm-5.0mm. The end of the overhang or extension **60** is the plane to which dilator **16** remains sub-planar until moving to tent and puncture the interatrial septum.

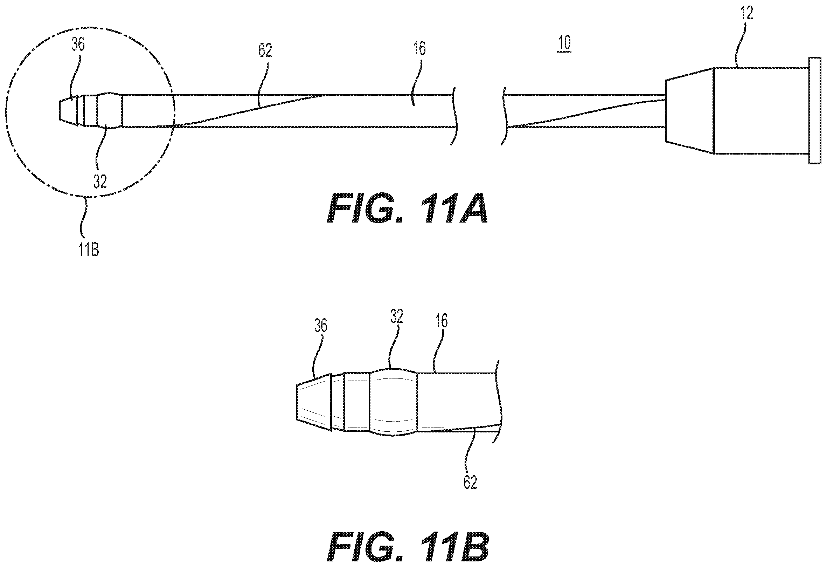

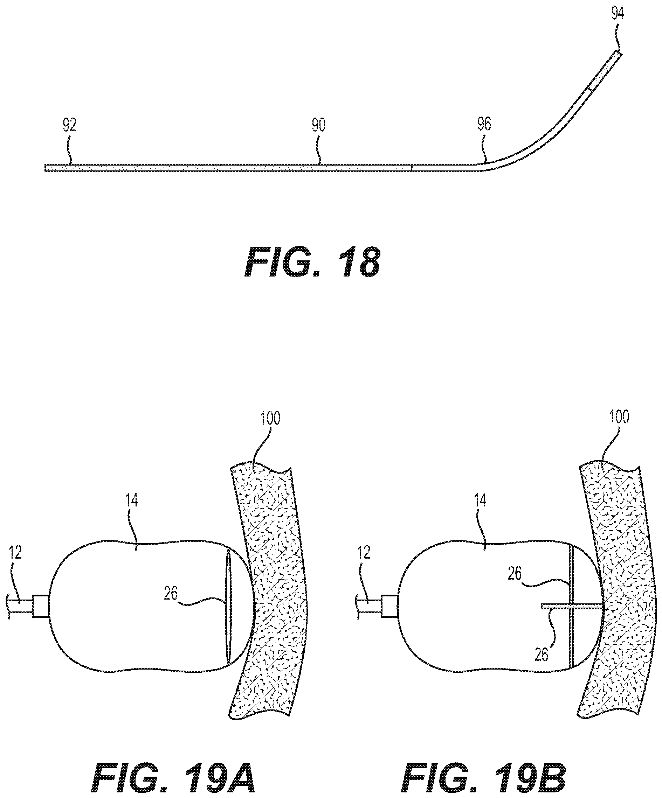

With reference now to FIGS. 11A and 11B, shown is a distal end of an embodiment of transseptal insertion device **10** with RF capability, with dilator **16** extended out from sheath **12** shaft. Dilator **16** includes a RF cap or tip **36** that may deliver RF energy for interatrial septum ablation purposes, as described above. RF cap **36** may be connected to RF energy source (not shown) at proximal end (not shown) of transseptal insertion device **10** with conductor **62.** Conductor **62** may wrap around shaft of dilator **16** as shown. Alternatively, conductor **62** may be extend through a lumen (not shown) of dilator **16** (*e.g*., such as lumen **56** shown in FIG. 18). Dilator **16** may include distal dilator seal **32** for sealing center lumen **15** (not shown) of sheath **12** and inflation ports **30** (not shown).

Variations of the above embodiments are within the scope of the invention. For example, the dilator shaft may have a preformed shape other than straight. The dilator shaft may contain a deflection apparatus. The electrode tip may be the distal dilator seal. The electrical conductor may wrap around the center lumen. Sheath or balloon shaft may contain a deflection apparatus.

Embodiments of transseptal insertion device **10** can successfully assist surgeons in carrying out at least one of the following techniques: visualization and stabilization of the intra atrial septum; visualization and stabilization of the fossa ovalis; and, guidance for transseptal puncture and across septum into safe zone of left atrium (away from structures such as aorta).

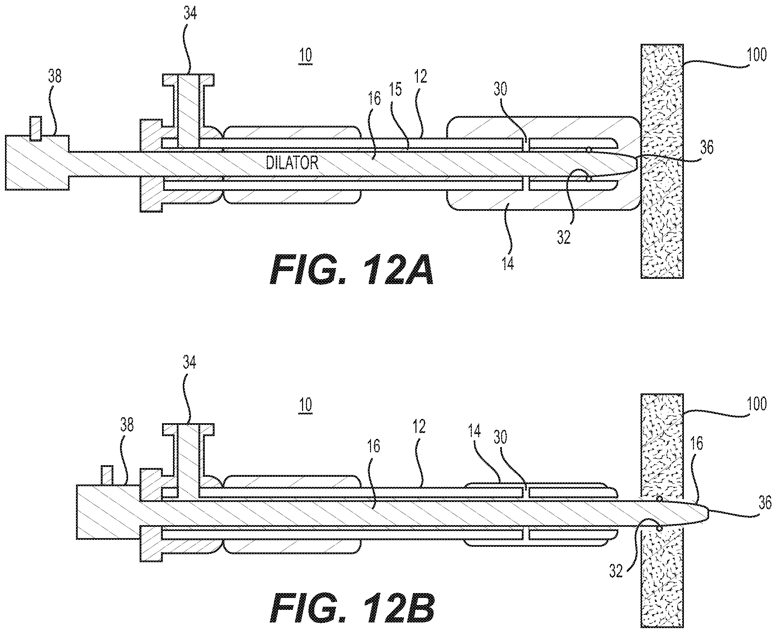

With reference now to FIGS. 12A-12B, shown is another embodiment of transseptal insertion device **10** that inflates overhanging balloon **14** using gas or fluid via sheath **12.** Embodiments include no separate port or hypotube for inflation or deflation of balloon **14.** Transseptal insertion device **10** may include inflation ports **30** in sheath **12** and dilator seal **32** on dilator **16.** Gas (*e.g*., air) or liquid is input into sheath **12** through inlet or port **34.** Gas or liquid exits sheath **12** through inflation ports **30,** inflating balloon **14** until fully inflated or inflated as much as desired. When dilator seal **32** is covered by inflated, overhanging sheath balloon **14,** as shown in FIG. 12A, dilator seal **32** is closed and balloon **14** remains inflated. As dilator **16** is advanced beyond overhanging balloon **14,** as shown in FIG. 12B, dilator seal **32** moves beyond the seal zone, dilator seal **32** is opened or unsealed, resulting in balloon **14** automatically and rapidly releasing inflation gas or fluid and deflating. The inflation gas or liquid exits the balloon **14** and the lumen **15** as noted above and is absorbed by the body and bloodstream. As such, inflation gas or liquid is inert and non-harmful. Inflation gas or fluid may include contrast agents enabling easier detection of inflation and deflation using imaging.

Embodiments of transseptal insertion device **10** include ablation tip **36,** *e.g*., radiofrequency (RF) ablation tip **36** (similar to crown or cap **22**) that may be used to deliver RF or other energy to ablate interaxial septum (septal wall) in order to puncture and create opening in septum. Transseptal insertion device **10** may include energy source at proximal end to deliver RF or other energy through dilator to ablation tip **36.** Energy source may be, *e.g.,* RF ablation connector on external hub **38.** Ablation connector on external hub **38** may be connected to proximal end of dilator **16,** as shown.

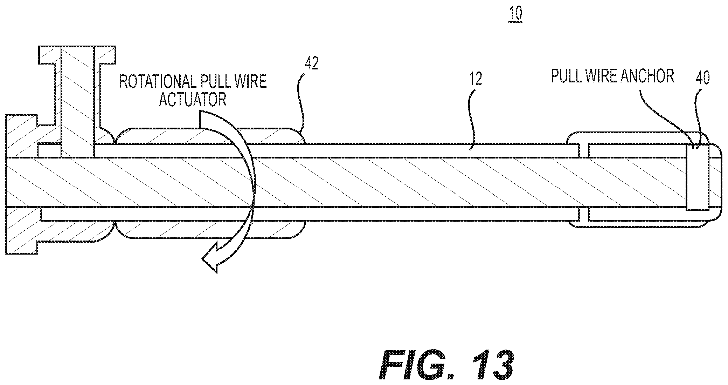

With reference now to FIG. 13, shown is an embodiment of transseptal insertion device **10** that includes a mechanical deflection mechanism. Mechanical deflection mechanism may enable distal end of sheath **12** to be deflected or angulated to various angles with respect to axis of transseptal insertion device **10.** Mechanical deflection mechanism may include a pull wire anchor **40** affixed to distal end of sheath **12** and pull wire actuator **42** connected to pull wire anchor **40** with pull wire (not shown). Rotation of pull wire actuator **42,** as shown, may exert force on pull wire anchor **40** that deflects or angulates distal end of sheath **12.** Pull wire actuator **42** may be rotated by handle connected thereto (not shown). Deflection or angulation of distal end of sheath **12** may enable better intersection (*e.g*., more perpendicular, flush) with interaxial septum and, therefore, better puncture and insertion by transseptal insertion device **10.**

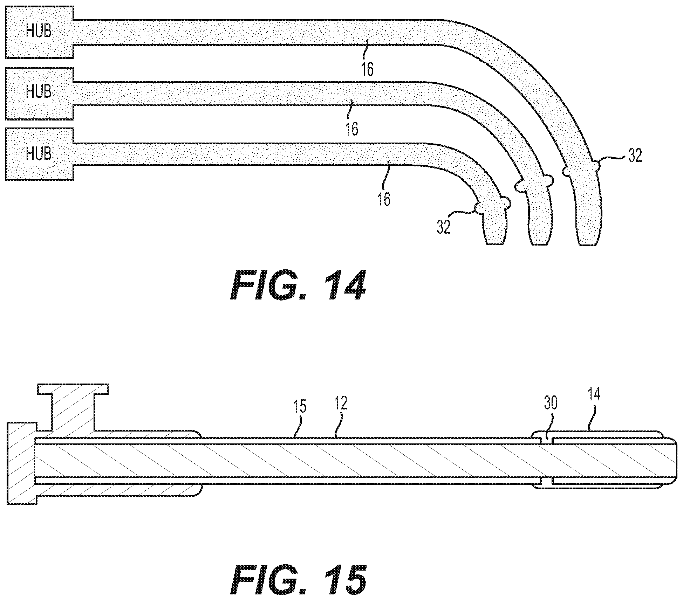

With reference now to FIG. 14, shown are three (3) embodiments of curved dilators **16,** each with a different curve profile (*i.e.,* different angle of deflection or curve). Curved dilators **16** may be used in embodiments of transseptal insertion device **10** with flexible or malleable sheath **12.** Such a flexible or malleable sheath **12** may be referred to as a steerable sheath **12** as it is 'steered" by curved dilator **16** inserted in sheath **12.**

With reference now to FIG. 15, shown is an embodiment of transseptal insertion device **10** that includes a steerable sheath **12.** In embodiment shown, distal end of sheath **12** is flexible or malleable so that sheath **12** may bend or angulate, *i.e.,* be steered, when, for example, curved dilator **16** is inserted. Proximal body of sheath **12** may be stiffened so that curved dilator **16** may be more easily pushed through sheath **12** when inserted therein.

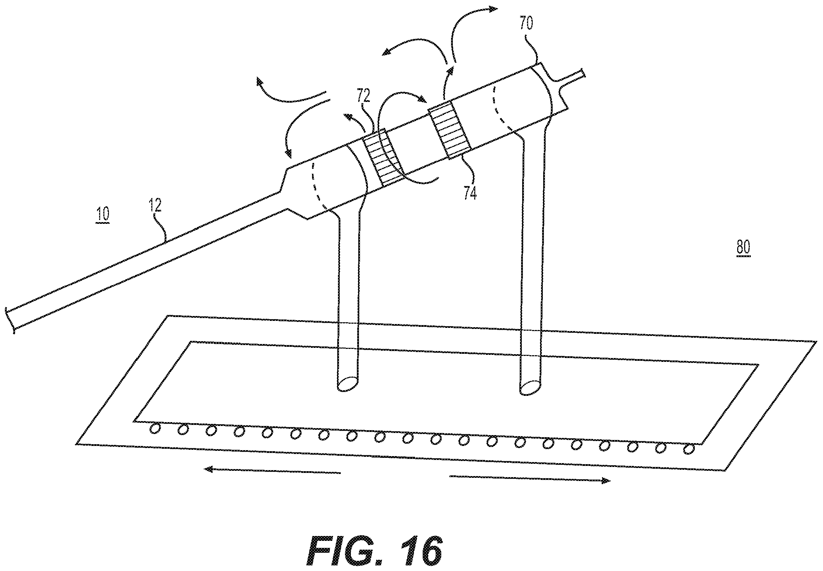

Embodiments of transseptal insertion device **10** may include a stabilizer in which the exterior of the catheter would be placed and which allows for very precise movements of the catheter. With reference now to FIG. 16, shown is an embodiment of transseptal insertion device/catheter **10** with an external stabilizer **80.** Stabilizer **80** keeps proximal end of transseptal insertion device **10** stable while allowing movement of transseptal insertion device **10** towards the distal and proximal ends of device **10,** rotational/torqueing movement of proximal end of device **10,** and manipulation of dials or other controls of device **10.** In effect, stabilizer **80** substantially prevents unwanted movement of the transseptal insertion device **10** and, importantly, distal end of sheath **12,** balloon **14,** and dilator **16.**

Stabilizer **80** includes connecting rods or arms **82** that connect stabilizer **80** to handle **70** at proximal end of transseptal insertion device **10.** Connecting arms **82** are attached to stabilizer platform **84.** Connecting arms **82** preferably hold the handle **70** securely and tightly, while permitting desired rotational movements and control manipulation. Stabilizer platform **84** is moveably attached to stabilizer base **86** so that stabilizer platform **84,** and hence handle **70** and transseptal insertion device **10,** may be slid forwards and backwards along axis of transseptal insertion device **10** towards and away from insertion point in patient (typically femoral vein at the groin of patient). Stabilizer base **86** is typically secured to a flat, stable surface, such as a table, or the leg of the patient. Configured as such, stabilizer **86** prevents unwanted vertical, rotational, or other movement of transseptal insertion device **10** and its handle **70,** keeping transseptal insertion device **10** and its handle **70** stable while permitting precise manipulation of handle **70** and its controls.

With continuing reference to FIG. 16, as shown, proximal end of transseptal insertion device **10** may include a handle **70** for control and manipulation of transseptal insertion device **10** and, particularly, dilator **16** and distal end of dilator **16.** Handle **70** may include a dial **72** that may be used to turn or deflect distal end of dilator **16,** effectively moving the distal end of dilator **16** up or down in relation to axis of transseptal insertion device **10** (as indicated by arrows in FIG. 16). Handle **70** may also include dial **74** for extruding/extending distal end of dilator **16** out of sheath **12** and retracting dilator **16** back into sheath **12,** effectively moving dilator **16** along axis of transseptal insertion device **10** (as indicated by arrows in FIG. 16). Handle **70** may also be rotated, as indicated by rotational arrow in FIG. 16, in order to deflect or turn distal end of transseptal insertion device to left or right in relation to axis of transseptal insertion device **10,** increasing or decreasing dilator **16** angle of deflection in that direction. If dial **72** moves distal end of dilator **16** along Y axis, and transseptal insertion device **10** axis is considered the Z axis, so that dial **74** moves dilator **16** along Z axis rotating handle **70** moves distal end of transseptal insertion device **10** (and hence distal end of dilator **16**) along X axis. Handle **70** includes port **76** to sheath lumen **15** through which dilator **16** and other devices inserted into transseptal insertion device **10** may be inserted. Handle **70** may also include one or more tubes or other ports permitting connection to external hubs and external energy sources, inflation liquids or gas,

In embodiments shown herein, balloon **14** and dilator **16** may be used as energy sources in the left atrium and may be used to deliver energy to the pulmonary veins, left atrial appendage, mitral valve and the left ventricle present in the left atrium. Such embodiments may include external energy sources connected to balloon **14** and/or dilator **16** through wires or other conductors extending lumen in sheath **12.** Delivery of energy via balloon **14** or dilator **16** may be thermal/Cryo or radiofrequency, laser or electrical. The delivery of such energy could be through a metallic platform such as a Nitinol cage inside or outside balloon **14.** Transseptal insertion device **10** may also include an energy source external to the proximal end of the sheath and operatively connected to balloon **14** to deliver energy to balloon **14.**

In other embodiments, balloon **14** may be re-inflated once distal tip **13** of transseptal insertion device **10** is passed through interatrial septum. Dilator **16** would be retracted into sheath **12** so that dilator seal **32** re-seals lumen **15** and inflation liquid or gas supplied through lumen **15** and into balloon **14.** In this manner, balloon **14** may be used as occlusion balloon in the left atrium, including in the pulmonary veins and left atrial appendage. In this manner, balloon **14** may be used for performing occlusion procedures in the left atrium.

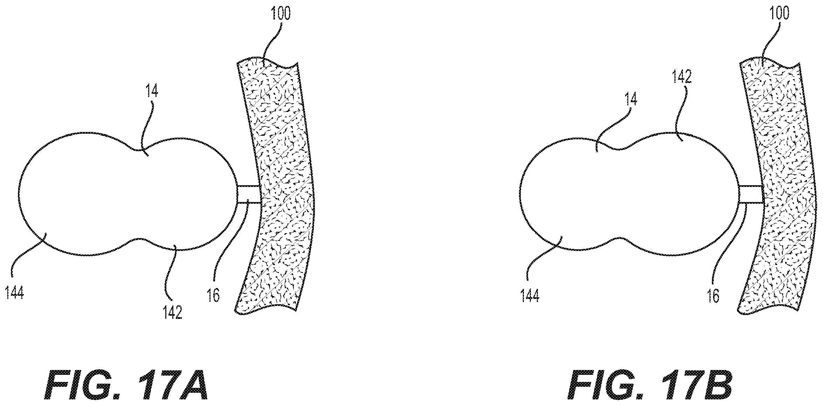

With reference now to FIGS. 17A and 17B shown is an embodiment of transseptal insertion device **10** enabling differential expansion of balloon **14.** Differential expansion of balloon **14** enables balloon **14** inflation to be adjusted based on the needs of the device operator and the conditions present in the patient's heart. For example, the size of the fossa ovalis portion of the interatrial septum may dictate the desired size of the inflated balloon **14** needed at the puncture site (interatrial septum if often punctured through the fossa ovalis). Fossae can vary greatly in size. The larger the fossa, the harder it will be to tent the interatrial septum with balloon **14.** Large fossa tend to be saggy and more difficult to manipulate. Hence, with a large fossa, a larger distal end of balloon **14** will make proper tenting of the interatrial septum easier. Indeed, it may be ideal to have balloon **14** inflated uniformly until intersecting or passing through fossa and then differentially expanding distal end **142** of balloon **14** to move fossa out of the way. In FIG. 17A, distal end or portion **142** of balloon **14** is smaller (less expanded) than proximal end **144** of balloon **14.**

Oppositely, the smaller the fossa, the easier it will be to tent the interatrial septum but, there will be less room to maneuver balloon **14** near interatrial septum. Consequently, a smaller distal end of balloon **14** is desired. It also may be beneficial to expand the proximal portion **144** more in order to help fix or secure balloon **14** in place. In FIG. 17B, distal end or portion of balloon **14** is larger (more expanded) than proximal end or portion of balloon **14.** In both FIGS. 17A and 17B, dilator **16** has extruded from sheath **12** and past distal end of balloon **14,** tenting interatrial septum **100,** and puncture is imminent.

This differential expansion of balloon **14** may be achieved, *e.g*., by using different materials for different portions of balloon **14** (*e.g.,* a more expandable material for distal end **142** than proximal end or portion **144,** or vice versa). In general, balloon **14** may be made of either compliant or non-compliant material, or a combination thereof. Compliant material will continue expanding as more inflating liquid or gas is added to balloon **14** (at least until failure). Non-compliant material will only inflate up to a set expansion or designated inflation level. Combinations of compliant and non-compliant material may be used to provide a differentially expanding balloon **14.** For example, distal end **142** may be formed from compliant material and proximal end **144** from non-compliant material to enable a larger distal end **142.** Oppositely, proximal end **144** may be formed from compliant material and distal end **142** from non-compliant material to enable a larger proximal end **144.** Other means for providing differential expansion of balloon **14** may be used, such as applying energy to different portions of balloon **14** to increase or decrease the compliance, and expandability, of that portion.

Balloon **14** may also be used to direct other equipment into these anatomical locations or be used as an angiographic or hemodynamic monitoring balloon. Differential expansion of balloon **14** may be utilized for proper orientation or direction of such equipment.

With reference now to FIG. 18, shown is an embodiment of a malleable transseptal needle **90** that may be used with transseptal insertion device **10** with a flexible sheath or otherwise capable of multiple angulations. In embodiments, malleable transseptal needle **90** may be of a variety of diameters and lengths. For example, embodiments include an 18 gauge transseptal needle and that is available in 71 cm, 89 cm, and 98 cm lengths. In embodiments, the malleable transseptal needle **90** has different stiffness in a proximal segment **92,** distal segment **94,** and in a middle segment **96** between. For example, malleable transseptal needle **90** may be stiffer in the proximal segment **92** and distal segment **94** and more flexible (less stiff) in a middle segment or mid-section **96.** The mid-section may be the section where the catheter **10** and dilator **16** angulate. In an embodiment, malleable transseptal needle **90** is used and a control handle provided that enables three-dimensional movements. Malleable transseptal needle **90** shown is, preferably, malleable or flexible at least in part. Proximal end **92** of malleable transseptal needle **90** may be stiff (*e.g.*, made from a stiff material, such as a metal). Mid-section or middle **96** of malleable transseptal needle **90** may be malleable or flexible (*e.g.*, made from a flexible, malleable material, such as rubber). Accordingly, mid-section may flex or bend, enabling malleable transseptal needle **90** to pass through angulated or flexed sheath **12.**

Distal end **94** of malleable transseptal needle **90** (*i.e.,* end that punctures interatrial cardiac septum) may be stiff with a cap or electrode at its tip for delivering energy to interatrial septum to puncture interatrial septum. In embodiments, transseptal needle is able to transmit radiofrequency energy to create a controlled septal puncture. Such a transseptal needle may or may not be malleable, but is able deliver RF energy through a cap or crown (*e.g*., an electrode) at its distal end tip. The needle **90** may be connected, *e.g*., on proximate end (not shown) to a radiofrequency (RF) energy source (not shown) at, *e.g*., external hub, that provides RF energy through needle to its distal end tip. In such an embodiment, dilator **16** may tent interaxial septum and RF energy capable transseptal needle may create puncture of interaxial septum through delivery of RF energy.

Embodiments of transseptal insertion device **10** may include a variety of additional features or components that improve its operation and enable increased control or additional functions. For example, the transseptal sheath **16** or balloon **14** may house (inside or on) an ultrasound chip or transducer which may be used to guide the insertion procedure. Ultrasound chip or transducer emits and receives ultrasound energy, that may be detected by known ultrasound visualization devices, to create an image of the cardiac chambers (*e.g.*, the right atrium, fossa, interatrial septum, left atrium, atrial appendage, mitral valve, ventricle, etc.). Ultrasound chips and transducers are transducers that convert ultrasound waves to electrical signals and/or vice versa. Those that both transmit and receive may also be called ultrasound transceivers; many ultrasound sensors besides being sensors are indeed transceivers because they can both sense and transmit. Such imaging will allow the operator(s) of transseptal insertion device **10** to visualize the cardiac chambers and the determine the location of the distal end or tip **13** of transseptal insertion device **10,** enabling more precise operation of transseptal insertion device **10.** Such a ultrasound chips or transducers used may be similar to ultrasound chip or transducer described in US Pat. App. Pub. 2003/019546, or any other ultrasound transducer known to those of ordinary skill in the art that may be fabricated on scale small enough to be deployed on or in sheath **16** or balloon **14.**

When deployed on balloon **14,** ultrasound chip or transducer may be forward facing (towards the left atrial septum) and deployed in the shape of a disc, a cross, or another rendition. So deployed, ultrasound chip or transducer enables visualization of the interatrial septum and the left atrial structures. Such an ultrasound chip may be connected to an imaging system either via a cable or wire that runs via sheath **12** or dilator **16** or connected via WiFi or other wireless connection.

With reference now to FIGS. 19A-19B, shown are embodiments of transseptal insertion device **10** with ultrasound imaging or visualizing capability. Balloon **14** shown includes one or more ultrasound chips or transducers **26** deployed in or on balloon **14.** Ultrasounds chips or transducers **26** may be ultrasound transceivers that both emit and receive waves, convert the ultrasound waves to electrical signals, transmit the electrical signals, *e.g*., through a wire that runs via sheath **12** or dilator **16** or connected via WiFi or other wireless connection, to an external imaging device that produces images from the received signals (both still and video images). Ultrasound chips or transducers **26** may be affixed to interior or exterior surface of balloon **14.** Ultrasound chips or transducers **26** may be arranged in a line, disc, or cross-shape. Ultrasound chips or transducers **26** may be arranged to be forward facing (*e.g*., on distal end of balloon facing towards interatrial septum), as shown in FIG. 19A, or in a different direction/orientation, such as sideways and forward facing (*e.g*., facing towards interatrial septum and facing perpendicular to the distal or front end), as shown in FIG. 19B. Indeed, orientation of ultrasound chips or transducers **26** may depend on whether balloon **14** is inflated or not. When balloon **14** is fully inflated, as shown in FIG. 19A, ultrasound transducer **26** may be forward facing (or forward and perpendicularly facing as shown in FIG. 19B). However, when balloon **14** is deflated, ultrasound transducer **26** may be folded flat and positioned on side of distal tip **13** of sheath **12** (as balloon **14** is shown in FIGS. 1A-1C). Hence, when balloon **14** is deflated, ultrasound chip or transducer **26** may be side-facing. During inflation ultrasound transducer **26** orientation will change as balloon **14** inflates (moving from side-facing orientation to forward facing orientation with the ultrasound transducer **26** shown in FIG. 19A). Accordingly, operator(s) of transseptal insertion device **10** may vary the inflation of balloon **14** to achieve different orientations of ultrasound transducer **26** for different imaging views.

Ultrasound chip or transducers **26** may emit and/or receive/detect ultrasound waves that may be reflect off of surfaces and structures, *e.g.*, within atrium, and then read by imaging system (not shown), *e.g*., connected to ultrasound chip or circuitry **26** via wire or cable extending through, *e.g*., lumen **15** in sheath **12.** In this manner, ultrasound chip or transducers **26** may enable visualization of the interatrial septum and the left atrial structures.

It is also noted that ultrasound chips or transducers **26** may be deployed on distal tip **13** of sheath **12** (or elsewhere on or in sheath **12**). Ultrasound chips or transducers **26** may be installed or configured to be forward facing (facing towards distal end of sheath **12**). Alternatively, ultrasound chips or transducers **26** may be flipped to be rear facing (facing towards proximal end of sheath **12**). Varying orientations of ultrasound chips or transducers **26** may be implemented.

Embodiments may include an additional balloon or dilator which would be able to dilate the distal end of sheath **12,** or the entire sheath length, thereby significantly increasing the French size of the sheath **12.** For example, balloons deployed within sheath **12** may be inflated to expand sheath **12.** In such embodiments, transseptal insertion device **10** may, therefore, be used to accommodate and deliver larger devices or be able to retrieve devices once they have been extruded from sheath **12** and have embolized. Such balloons may be inflated through

In embodiments, energy, typically electrical energy, may directed through transseptal insertion device **10** may be used to increase or decrease the French size of sheath **12.** In such embodiments, sheath **12** is fabricated from materials that are known to increase in malleability and or expand when certain energies are applied. In this manner, the French size of sheath **12** may be adjusted to a size deemed necessary during a given procedure. Such energy may be applied through wires or conductive material, connected to energy source external to proximal end of transseptal insertion device **10,** attached to or fabricated within sheath **12** or other components of transseptal insertion device **10.** Likewise, parts or portions of transseptal insertion device **10** may be selectively made more rigid or more malleable/soft with the application of energy. Therefore, with the application of differential energy to different parts of transseptal insertion device **10** at different times, transseptal insertion device **10** size may be adjusted to enable various devices that are ordinarily larger and bulkier than the catheter to traverse through the catheter. In embodiments, transseptal insertion device **10** may accommodate devices up to 36 Fr.

In an embodiment of transseptal insertion device **10,** visualization of an intrathoracic region of interest using MRI techniques may be provided. Embodiments may, for example, provide a needle system comprising a hollow needle having a distal portion and a proximal portion, said distal portion having a distal-most end sharpened for penetrating a myocardial wall. The needle may include a first conductor, an insulator/dielectric applied to cover the first conductor over the proximal portion of said needle and a second conductor applied to cover the insulator/dielectric. The method may further direct the needle system into proximity to a myocardial wall, track progress of the needle system using active MRI tracking, penetrate the myocardial wall to approach the intrathoracic region of interest, and, use the needle system as an MRI antenna to receive magnetic resonance signals from the intrathoracic region of interest.

In related embodiments, MRI antenna may be installed on distal tip **13** of sheath **12,** dilator **16** or on balloon **14,** similar to ultrasound chips or transducers **26** described above. Wires connecting such MRI antenna or other MRI components may pass through lumen in dilator **16** or sheath **12** and connect with appropriate magnetic resonance energy source on exterior of distal end of transseptal insertion device **10.**

Since many modifications, variations, and changes in detail can be made to the described preferred embodiments of the invention, it is intended that all matters in the foregoing description and shown in the accompanying drawings be interpreted as illustrative and not in a limiting sense. Consequently, the scope of the invention should be determined by the appended claims.

## Claims

1. A transseptal insertion device (10) which is suitable for facilitating precise and safe transseptal puncture of a cardiac interatrial septum (100), comprising:
a sheath (12) that defines at least one lumen (15) therein and has a distal end that is closest to the cardiac interatrial septum (100) of a patient when the transseptal insertion device is in use and a proximal end that is external to the patent;
a balloon (14) that is connected to the distal end of the sheath (12), wherein:
the balloon (14), when inflated and the transseptal insertion device (10) is in use, overhangs and extends past the distal end of the sheath, preventing accidental puncturing of the cardiac interatrial septum (100) and stabilizing the transseptal insertion device against fossa ovalis of the cardiac interatrial septum;
and a dilator (16) that is positioned within the at least one lumen (15) when the transseptal insertion device (10) is in use, wherein the dilator (16) has a distal end and is designed to and is capable of precisely puncturing the cardiac interatrial septum (100), **characterised in that** the balloon includes one or more ultrasound transceivers that emit and receive ultrasound waves, convert the ultrasound waves to electrical signals, and transmit the electrical signals to an external imaging device that produces images of the cardiac interatrial septum from the received signals,
wherein the one or more ultrasound transceivers (26) are located on the balloon surface,
wherein the one or more ultrasound transceivers (26) are oriented towards the cardiac interatrial septum (100) when the balloon (14) is fully inflated and the distal end of the sheath (12) is oriented towards the cardiac interatrial septum (100).

2. The transseptal insertion device (10) of claim 1 wherein the one or more ultrasound transceivers (26) are configured in the shape of a cross so that one or more ultrasound transceivers are oriented towards the cardiac interatrial septum (100) when the balloon (14) is fully inflated and the distal end of the sheath (12) is oriented towards the cardiac interatrial septum (100) and perpendicular to the sheath (12) or wherein the one or more ultrasound transceivers (26) are configured in the shape of a disc.

3. The transseptal insertion device (10) of claim 1 wherein the one or more ultrasound transceivers (26) are connected to an external imaging device through a wire that runs via lumen in sheath (12) or wherein the one or more ultrasound transceivers (26) are connected to an external imaging device via WiFi or other wireless connection.

4. A transseptal insertion device (10) which is suitable for facilitating precise and safe transseptal puncture of a cardiac interatrial septum (100), comprising:
a sheath (12) that defines at least one lumen (15) therein and has a distal end that is closest to the cardiac interatrial septum (100) of a patient when the transseptal insertion device is in use and a proximal end that is external to the patent;
a balloon (14) that is connected to the distal end of the sheath (12), wherein:
the balloon (14), when inflated and the transseptal insertion device (10) is in use, overhangs and extends past the distal end of the sheath, preventing accidental puncturing of the cardiac interatrial septum (100) and stabilizing the transseptal insertion device against fossa ovalis of the cardiac interatrial septum;
and a dilator (16) that is positioned within the at least one lumen (15) when the transseptal insertion device (10) is in use, wherein the dilator has a distal end and is designed to and is capable of precisely puncturing the cardiac interatrial septum (100), **characterised in that**
the balloon (14) is constructed of compliant and non-compliant material so that different portions of the balloon will expand differentially when the balloon is inflated;
and **in that** the device further comprises one or more ultrasound transceivers (26) that emit and receive ultrasound waves, convert the ultrasound waves to electrical signals, and transmit the electrical signals to an external imaging device that produces images of the cardiac interatrial septum (100) from the received signals, wherein the one or more ultrasound transceivers (26) are located on the balloon (14),
wherein the one or more ultrasound transceivers (26) are oriented towards the cardiac interatrial septum (100) when the balloon (14) is fully inflated and the distal end of the sheath (12) is oriented towards the cardiac interatrial septum (100).

5. The transseptal insertion device of claim 4 wherein the one or more ultrasound transceivers are located on the dilator.

## Patentansprüche

1. Vorrichtung (10) zur transseptalen Einführung, die zur Erleichterung der präzisen und sicheren transseptalen Punktion eines Vorhofseptums (100) des Herzens geeignet ist, umfassend:
eine Hülse (12), die mindestens ein Lumen (15) darin definiert und ein distales Ende, das sich am nächsten zu dem Vorhofseptum (100) des Herzens eines Patienten befindet, wenn die Vorrichtung zur transseptalen Einführung in Gebrauch ist, und ein proximales Ende aufweist, das sich außerhalb des Patienten befindet;
einen Ballon (14), der mit dem distalen Ende der Hülse (12) verbunden ist, wobei:
der Ballon (14), wenn er befüllt ist und die Vorrichtung (10) zur transseptalen Einführung in Gebrauch ist, überhängt und sich über das distale Ende der Hülse hinaus erstreckt, wodurch versehentliche Punktion des Vorhofseptums (100) des Herzens verhindert wird und die Vorrichtung zur transseptalen Einführung gegen die Fossa ovalis des Vorhofseptums des Herzens stabilisiert wird; und
einen Dilator (16), der innerhalb des mindestens einen Lumens (15) positioniert ist, wenn die Vorrichtung (10) zur transseptalen Einführung in Gebrauch ist, wobei der Dilator (16) ein distales Ende hat und konzipiert und imstande ist, das Vorhofseptum (100) des Herzens präzise zu punktieren, **dadurch gekennzeichnet, dass** der Ballon einen oder mehrere Ultraschallsendeempfänger einschließt, die Ultraschallwellen emittieren und empfangen, die Ultraschallwellen in elektrische Signale konvertieren und die elektrischen Signale an eine externe Bildgebungsvorrichtung übermitteln, die aus den empfangenen Signalen Bilder des Vorhofseptums des Herzens produziert,
wobei der eine oder die mehreren Ultraschallsendeempfänger (26) sich auf der Ballonoberfläche befinden,
wobei der eine oder die mehreren Ultraschallsendeempfänger (26) in Richtung des Vorhofseptums (100) des Herzens orientiert ist/sind, wenn der Ballon (14) vollständig befüllt ist, und das distale Ende der Hülse (12) in Richtung des Vorhofseptums (100) des Herzens orientiert ist.

2. Vorrichtung (10) zur transseptalen Einführung nach Anspruch 1, wobei der eine oder die mehreren Ultraschallsendeempfänger (26) in Form eines Kreuzes ausgestaltet ist/sind, so dass ein oder mehrere Ultraschallsendeempfänger in Richtung des Vorhofseptums (100) des Herzens orientiert ist/sind, wenn der Ballon (14) vollständig befüllt ist und das distale Ende der Hülse (12) in Richtung des Vorhofseptums (100) des Herzens und senkrecht zu der Hülse (12) orientiert ist, oder wobei der eine oder die mehreren Ultraschallsendeempfänger (26) in Form einer Scheibe ausgestaltet ist/sind.

3. Vorrichtung (10) zur transseptalen Einführung nach Anspruch 1, wobei der eine oder die mehreren Ultraschallsendeempfänger (26) über einen Draht, der mittels des Lumens in der Hülse (12) läuft, mit einer externen Bildgebungsvorrichtung verbunden ist/sind, oder wobei der eine oder die mehreren Ultraschallsendeempfänger (26) über WiFi oder sonstige drahtlose Verbindung mit einer externen Bildgebungsvorrichtung verbunden ist/sind.

4. Vorrichtung (10) zur transseptalen Einführung, die zur Erleichterung der präzisen und sicheren transseptalen Punktion eines Vorhofseptums (100) des Herzens geeignet ist, umfassend:
eine Hülse (12), die mindestens ein Lumen (15) darin definiert und ein distales Ende, das sich am nächsten zu dem Vorhofseptum (100) des Herzens eines Patienten befindet, wenn die Vorrichtung zur transseptalen Einführung in Gebrauch ist, und ein proximales Ende aufweist, das sich außerhalb des Patienten befindet;
einen Ballon (14), der mit dem distalen Ende der Hülse (12) verbunden ist, wobei:
der Ballon (14), wenn er befüllt ist und die Vorrichtung (10) zur transseptalen Einführung in Gebrauch ist, überhängt und sich über das distale Ende der Hülse hinaus erstreckt, wodurch versehentliche Punktion des Vorhofseptums (100) des Herzens verhindert wird und die Vorrichtung zur transseptalen Einführung gegen die Fossa ovalis des Vorhofseptums des Herzens stabilisiert wird; und
einen Dilator (16), der innerhalb des mindestens einen Lumens (15) positioniert ist, wenn die Vorrichtung (10) zur transseptalen Einführung in Gebrauch ist, wobei der Dilator ein distales Ende hat und konzipiert und imstande ist, das Vorhofseptum (100) des Herzens präzise zu punktieren, **dadurch gekennzeichnet, dass** der Ballon (14) aus nachgiebigem und nicht-nachgiebigem Material aufgebaut ist, so dass unterschiedliche Anteile des Ballons unterschiedlich expandieren, wenn der Ballon befüllt wird; und
die Vorrichtung des Weiteren umfasst:
einen oder mehrere Ultraschallsendeempfänger (26), die Ultraschallwellen emittieren und empfangen, die Ultraschallwellen in elektrische Signale konvertieren und die elektrischen Signale an eine externe Bildgebungsvorrichtung übermitteln, die aus den empfangenen Signalen Bilder des Vorhofseptums (100) des Herzens produziert, wobei der eine oder die mehreren Ultraschallsendeempfänger (26) sich auf dem Ballon (14) befinden,
wobei der eine oder die mehreren Ultraschallsendeempfänger (26) in Richtung des Vorhofseptums (100) des Herzens orientiert sind, wenn der Ballon (14) vollständig befüllt ist, und das distale Ende der Hülse (12) in Richtung des Vorhofseptums (100) des Herzens orientiert ist.

5. Vorrichtung zur transseptalen Einführung nach Anspruch 4, wobei der eine oder die mehreren Ultraschallsendeempfänger sich auf dem Dilator befindet/befinden.

## Revendications

1. Dispositif d'insertion transseptale (10) qui est approprié pour faciliter une ponction transseptale précise et sûre d'un septum interatrial cardiaque (100), comprenant :
une gaine (12) qui définit au moins une lumière (15) en son sein et possède une extrémité distale qui est la plus proche du septum interatrial cardiaque (100) d'un patient lorsque le dispositif d'insertion transseptale est en cours d'utilisation, et une extrémité proximale qui est extérieure au patient ;
un ballonnet (14) qui est relié à l'extrémité distale de la gaine (12), dans lequel :
le ballonnet (14), lorsqu'il est gonflé et que le dispositif d'insertion transseptale (10) est en cours d'utilisation, surplombe l'extrémité distale de la gaine et s'étend au-delà de celle-ci, empêchant la ponction accidentelle du septum interatrial cardiaque (100) et stabilisant le dispositif d'insertion transseptale contre la fosse ovale du septum interatrial cardiaque ; et un dilatateur (16) qui est positionné à l'intérieur de l'au moins une lumière (15) lorsque le dispositif d'insertion transseptale (10) est en cours d'utilisation, dans lequel le dilatateur (16) possède une extrémité distale et est conçu pour, et est susceptible de, ponctionner précisément le septum interatrial cardiaque (100), **caractérisé en ce que**
le ballonnet comprend un ou plusieurs émetteurs-récepteurs ultrasonores qui émettent et reçoivent des ondes ultrasonores, convertissent les ondes ultrasonores en signaux électriques et émettent les signaux électriques vers un dispositif d'imagerie externe qui produit des images du septum interatrial cardiaque à partir des signaux reçus,
dans lequel les un ou plusieurs émetteurs-récepteurs ultrasonores (26) sont situés sur la surface du ballonnet,
dans lequel les un ou plusieurs émetteurs-récepteurs ultrasonores (26) sont orientés vers le septum interatrial cardiaque (100) lorsque le ballonnet (14) est entièrement gonflé et que l'extrémité distale de la gaine (12) est orientée vers le septum interatrial cardiaque (100).

2. Dispositif d'insertion transseptale (10) selon la revendication 1 dans lequel les un ou plusieurs émetteurs-récepteurs ultrasonores (26) sont configurés en forme de croix de sorte qu'un ou plusieurs émetteurs-récepteurs ultrasonores soient orientés vers le septum interatrial cardiaque (100) lorsque le ballonnet (14) est complètement gonflé et que l'extrémité distale de la gaine (12) est orientée vers le septum interatrial cardiaque (100) et perpendiculaire à la gaine (12) ou dans lequel les un ou plusieurs émetteurs-récepteurs ultrasonores (26) sont configurés sous la forme d'un disque.

3. Dispositif d'insertion transseptale (10) selon la revendication 1 dans lequel les un ou plusieurs émetteurs-récepteurs ultrasonores (26) sont connectés à un dispositif d'imagerie externe par le biais d'un fil qui parcourt la lumière dans la gaine (12) ou dans lequel les un ou plusieurs émetteurs-récepteurs ultrasonores (26) sont connectés à un dispositif d'imagerie externe par WiFi ou une autre connexion sans fil.

4. Dispositif d'insertion transseptale (10) qui est approprié pour faciliter une ponction transseptale précise et sûre d'un septum interatrial cardiaque (100), comprenant :
une gaine (12) qui définit au moins une lumière (15) en son sein et possède une extrémité distale qui est la plus proche du septum interatrial cardiaque (100) d'un patient lorsque le dispositif d'insertion transseptale est en cours d'utilisation, et une extrémité proximale qui est extérieure au patient ;
un ballonnet (14) qui est relié à l'extrémité distale de la gaine (12), dans lequel :
le ballonnet (14), lorsqu'il est gonflé et que le dispositif d'insertion transseptale (10) est en cours d'utilisation, surplombe l'extrémité distale de la gaine et s'étend au-delà de celle-ci, empêchant la ponction accidentelle du septum interatrial cardiaque (100) et stabilisant le dispositif d'insertion transseptale contre la fosse ovale du septum interatrial cardiaque ; et un dilatateur (16) qui est positionné à l'intérieur de l'au moins une lumière (15) lorsque le dispositif d'insertion transseptale (10) est en cours d'utilisation, dans lequel le dilatateur (16) possède une extrémité distale et est conçu pour, et est susceptible de, ponctionner précisément le septum interatrial cardiaque (100), **caractérisé en ce que**
le ballonnet (14) est constitué d'un matériau conforme et non conforme de sorte que différentes parties du ballonnet se dilatent de manière différentielle lorsque le ballonnet est gonflé ;
et **en ce que** le dispositif comprend en outre
un ou plusieurs émetteurs-récepteurs ultrasonores (26) qui émettent et reçoivent des ondes ultrasonores, convertissent les ondes ultrasonores en signaux électriques, et émettent les signaux électriques vers un dispositif d'imagerie externe qui produit des images du septum interatrial cardiaque (100) à partir des signaux reçus, dans lequel les un ou plusieurs émetteurs-récepteurs ultrasonores (26) sont situés sur le ballonnet (14),
dans lequel les un ou plusieurs émetteurs-récepteurs ultrasonores (26) sont orientés vers le septum interatrial cardiaque (100) lorsque le ballonnet (14) est entièrement gonflé et que l'extrémité distale de la gaine (12) est orientée vers le septum interatrial cardiaque (100).

5. Dispositif d'insertion transseptale selon la revendication 4, dans lequel les un ou plusieurs émetteurs-récepteurs ultrasonores sont situés sur le dilatateur.
